# EUROPEAN PATENT SPECIFICATION

(11) **EP 0 567 234 B1**
(45) Date of publication and mention of the grant of the patent: **30.12.1998**
(21) Application number: 93302317.8
(22) Date of filing: 25.03.1993
(51) Int. Cl.: A61K 47/42, A61K 9/16

(54) **Biopolymer composites**
Biopolymerverbundmaterial
Matière composite contenant un biopolymère

(30) Priority: 25.03.1992 GB 9206508
(43) Date of publication of application: 27.10.1993
(73) Proprietor: JOHNSON & JOHNSON MEDICAL, INC., Arlington, Texas 76014 (US)
(72) Inventor: Haynes, Carla A., Cambuslang, Glasgow, G72 8NH (GB); Harvey, Wilson, Gargunnock, Stirling, FK8 3AX (GB); Watt, Paul W., Broomridge, Stirling, FK7 7TR (GB)
(74) Representative: Fisher, Adrian John

(56) References cited:
- FR-M- 6 652

## Description

This invention relates to composites of an insoluble protein matrix with an oleaginous component, and to the preparation of such composites. Such composites are useful in a variety of applications, and particularly as materials for surgical dressings and biomedical implants, and as cosmetic materials for application to the skin.

Biopolymers, such as animal proteins and plant polysaccharides, have been used in recent years in a number of diverse applications, including biomedical applications. For example, alginates (which are polysaccharides derived from seaweeds) are valuable for their haemostatic properties, while collagen (the major protein of skin and connective tissue) has been used in wound dressing materials, as well as in surgical sponges (see, for example, US-A-3632361; US-A-4412947; US-A-3823212; Chvapil, J. Biomed. Mater. Res. 11, 721 to 741 (1977); Huc, J. Am. Leather Chem. Assoc. 80, 195 to 212 (1985); and GB-A-2058084). Collagen is attractive in a biomedical context, principally because it is biocompatible, resorbable, structurally versatile and also has haemostatic properties.

Collagen sponges are known to be capable of absorbing large quantities of water. However, it has also been suggested in WO-A-9101945 that sponges of collagen or of certain other natural products can be used to absorb oils. In particular, it is suggested that such sponges can be used to separate and recover oils from aqueous media, and this is said to be of utility in treating offshore oil spills.

According to WO-A-9101945, collagen and gelatin sponges may be capable of absorbing in excess of 50 times their own weight of oil, and the absorbed oil may be recovered by squeezing or by other physical compression means. It is therefore believed that the absorbed oil is held predominantly or exclusively within the pores of the sponge.

Japanese laid-open patent application JP-A-55084167 (Lion Hamigaki KK) describes medicated sponge bands for the treatment of periodontal disease. The sponge bands comprise a sponge matrix of a soluble polymer having droplets of non-volatile oil dispersed therein. The non-volatile oil may contain dissolved medicaments. The sponge bands are formed by freeze drying an oil-in-water emulsion having the soluble polymer dissolved in the aqueous phase. The soluble polymers may be natural, synthetic or semi-synthetic polymers such as cellulose derivatives, natural gums, sodium alginate, gelatin or polyvinylpyrrolidone. In use, the medicated sponge bands are applied to affected areas such as mucous membranes in the oral cavity. The bands rapidly absorb water to form a sticky oil-in-water ointment. The sponge band is normally provided with an impermeable backing layer to improve the persistence of the ointment at the affected area.

The present invention is based on the discovery that a biopolymer matrix based on an insoluble protein can be formed with significant quantities of an oleaginous material held within the matrix itself (rather than physically entrapped within the pores of such a matrix), and that such a material exhibits a surprisingly non-oily or non-greasy appearance and feel.

According to the present invention, there is provided a process for preparing a composite comprising a matrix of an insoluble protein and an oleaginous material, said process comprising the steps of mixing a protein, the oleaginous material and water to form an emulsion of said oleaginous material in an aqueous dispersion of the protein and subsequently drying the emulsion.

In one embodiment, the emulsion is dried as a thin layer to form a film. The invention thus further provides a film comprising a composite of a matrix of an insoluble protein and an oleaginous material, the oleaginous material being distributed throughout the film as discrete microscopic droplets. Typically, the droplets have a maximum dimension less than 25 µm, and generally in the range 0.5 to 10 µm.

Drying to form a film may conveniently be carried out at a temperature of from 1°C to 100°C, and more preferably at a temperature of from 15° to 60°C. In this embodiment, the emulsion preferably also includes a plasticiser to facilitate film formation. Suitable plasticisers include glycerol, sorbitol and polyethylene glycol, which will generally be used in amounts up to 40% by weight of the film. More preferably, the plasticiser constitutes from 5 to 30% by weight of the film. The film may optionally be perforated or reticulated.

In an alternative embodiment, the emulsion is frozen and then freeze dried, to form a sponge, the matrix of the sponge being formed of the insoluble protein/oleaginous material composite. In this embodiment, too, the oleaginous material may appear as discrete microscopic droplets when surface of the sponge matrix is viewed.

In yet other embodiments the composite is heteromorphic. That is to say, the composite is structurally and/or compositionally inhomogeneous. For example, a heteromorphic composite may be made by mixing flakes of a composite film according to the present invention into the oil-in-water emulsion followed by freeze drying the emulsion. This procedure results in a heteromorphic sponge having flakes of film composite embedded in a sponge matrix. Likewise, it is possible to make a laminated heteromorphic sponge having alternating layers of composite film and composite sponge.

In all of the above-described embodiments, the emulsion is preferably degassed to removed trapped air before it is dried.

The oleaginous material may be an oil, a grease, a fat or a wax. Preferably, the mixing of the protein, oleaginous material and water is carried out at a temperature at which the oleaginous material is liquid. Generally, the oleaginous material will be an oil at room temperature (eg. at 25°C). Suitable oils include mineral oils and vegetable oils. In the case of composite films according to the invention, the oleaginous material is preferably a dispersible oil, such as Labrafil M2125 CS, manufactured by Gattefossé s.a., 36 chemin de Genas, 69800 Saint-Priest, France. Labrafil M2125 CS is a mixture of unsaturated polyglycolysed glycerides obtained by partial alcoholysis of maize oil. Films formed using such an oil according to the present invention have been found to give reduced shrinkage on drying, as compared with films formed using conventional oils.

Preferably, the protein used to form the aqueous dispersion is itself an insoluble fibrous protein. The aqueous dispersion is then an aqueous suspension of insoluble protein fibres, and drying the emulsion results directly in the composite having a matrix of insoluble protein.

In alternative embodiments, the protein is a water soluble protein such as gelatin and the aqueous dispersion is a solution of that protein. These embodiments then further comprise the step of adding a cross-linking agent such as HMDI (hexamethylene diisocyanate), water soluble carbodiimide or glutaraldehyde to the emulsion to cross-link the soluble protein and render it insoluble in the finished composite.

Suitable insoluble fibrous proteins preferred for the process of the invention may include the so-called structural fibrous proteins and derivatives thereof, such as insoluble collagen, keratin, fibrin and elastin.

Preferably, the insoluble fibrous protein is predominantly comprised of insoluble collagen, which may suitably be obtained from bovine skin. Such collagen preferably has a fibre length of from 0.005 to 5 mm, and more preferably from 0.01 to 3 mm. Conveniently, but not essentially, the collagen is swollen prior to use, either in acid or in alkali. Acid swelling is preferred, with optimum swelling occurring in the pH range 2 to 3.5. Organic acids (e.g. acetic acid, malic acid, lactic acid and citric acid) and mineral acids (e.g. hydrochloric acid and sulphuric acid) can be used, but organic acids are preferable since they facilitate greater swelling of the collagen.

In general, the insoluble fibrous protein content of the emulsion is in the range 0.01 to 10% w/v, and preferably in the range 0.3 to 5% w/v.

The ratio of oleaginous material to protein in the emulsion (and hence in the final product) depends upon the end use to which the product is to be put, and also on the physical form of the end product. If the product is to be used in the form of a sponge, the weight ratio of oleaginous material to insoluble protein is preferably in the range 0.001:1 to 100:1 and more preferably in the range 0.001:1 to 25:1. Still more preferably the weight ratio of oleaginous material to insoluble protein is in the range from 0.05:1 to 25:1.

If the product is to be used in the form of a film, the weight ratio of oleaginous material to insoluble fibrous protein is preferably in the range 0.001:1 to 20:1, more preferably from 0.001:1 to 10:1 and most preferably from 0.001:1 to 5:1.

It will be understood that composites according to the invention may contain both insoluble and soluble proteins and other, additional biopolymers such as polysaccharides. The maximum amount of oleaginous material which it is appropriate to employ may depend on the total amount of protein in the mixture.

Cross-linking agents, such as HMDI (hexamethylene diisocyanate), water soluble carbodiimide or glutaraldehyde, can also be added during the emulsion manufacture to cross link the insoluble fibrous protein, soluble protein or soluble polysaccharides present in the emulsion thus increasing the tensile strength of the resulting matrix.

Composites according to the present invention containing low quantities of oleaginous material are conveniently made by dissolving the oleaginous material in a volatile organic solvent, such as n-hexane, prior to forming the emulsion. This aids the dispersion of the oleaginous material throughout the matrix. The volatile solvent is then lost during the drying process leaving the oleaginous material dispersed as microdroplets throughout the matrix.

An emulsifier may sometimes be incorporated in the emulsion to produce a more homogeneous dispersion of oleaginous material throughout the protein matrix. However, the use of pepsin solubilized collagen, gelatin or a polysaccharide in addition to the insoluble protein generally avoids the need for a separate emulsifier, and this is preferred for biomedical applications. For cosmetic applications, a wide range of commercially available emulsifiers may be used, such as lecithins, mono and diglycerides of fatty acids, and sorbitan esters.

It has also been found, surprisingly, that insoluble fibrous proteins are effective emulsifiers in aqueous suspension. Stable oil-in-water emulsions can be prepared for example by homogenising aqueous suspensions of insoluble protein fibres such as insoluble collagen fibres with oil at high shear to produce microdroplets of the oil dispersed between the protein fibres. The collagen fibres may be acid-swollen as described above. The insoluble protein content of the emulsions can range from 0.5% to 15% by weight of the emulsion, but is preferably from 1% to 10% by weight. The oil content can range from 1% to 50% by weight of the emulsion but is preferably 10% to 35% by weight.

When a separate emulsifier is used to form the emulsions according to the present invention, it is preferably used in the emulsion in an amount from 0.01 to 10% w/v. It will be understood, however, that the precise amount of emulsifier used will depend on the amount of oleaginous material used, the type of emulsifier, and the overall total dilution.

The composites of the present invention may be used as controlled release vehicles for pharmaceutically active agents. For this purpose, the composite may be applied as a wound dressing or as an implant. The pharmaceutically active agents which may be employed include hydrophobic agents and hydrophilic agents.

Hydrophobic pharmaceutically active agents, which may be dissolved in the oleaginous phase, include steroids (such as testosterone and oestradiol) and retinol. Hydrophilic pharmaceutically active agents include antibiotics (such as penicillins and cephalosporins), antiseptics (such as chlorhexidine), β-blockers (such as propanolol), and peptide hormones and growth factors. In some cases, these may be suspended in the oleaginous material (e.g. in particulate, crystalline form), and release of the agent in this case will depend on partitioning into the "aqueous" phase, i.e. the insoluble protein matrix. In other cases, the hydrophilic agents may be incorporated directly into the insoluble protein matrix, when the rate of their release is modified by the presence, of droplets of the oleaginous material. A preferred such hydrophilic active agent is micronised mannose-6-phosphate.
In a further alternative embodiment, the emulsion is formed into beads or microspheres. This can be achieved by freezing droplets of the emulsion in liquid nitrogen and lyophilising as described in US Patent 4,837,285. Alternatively, microspheres can be prepared by forming a dispersion of the oil in water biopolymer emulsion in a water immiscible solvent/organic phase. Following addition of a cross linking agent, the biopolymer/oil emulsion particles are separated from the continuous solvent phase and the particles are dried, preferably by lyophilisation as described in either WO 9210287 or WO 9106286.

The composites of the invention are particularly useful as wound dressings or implants or as dressings specifically for the treatment of burns. The hydrophobic nature of the material (the degree of hydrophobicity is dependant on the content of the oleaginous phase) may be used to reduce moisture loss from wounds, to reduce trauma on removal, or to deliver active agents to the wound site. In these wound dressings or implants, factors which may promote wound healing can be incorporated into the matrix, these include growth factors, glycosaminoglycans (GAGS) such as hyaluronic acid, chondroitin sulphate or the low molecular weight heparins. Furthermore additional factors which have potential to reduce wound scarring such as mannose-6-phosphate, TGF-β₃, and anti TGF β ₁ and β ₂ can be dissolved/suspended in either the hydrophobic or hydrophilic phases of these matrices.

An additional application for the insoluble protein/oil films is as medicated implants for the treatment of periodontitis. As previously mentioned, these films can incorporate either hydrophobic or hydrophilic active agents. By controlling the quantity of the oleaginous material and the size and heterogeneity of the oil micro droplets, the delivery of both types of actives can be manipulated and controlled.

Furthermore, although not essential, the insoluble protein/oil films can be reinforced by incorporating a biodegradable or non-biodegradable mesh. This supporting mesh can be, for example, and oxidised regenerated cellulose mesh such as Surgicel™ or a polylactate polyglycolate mesh such as Vicryl™. Incorporation of this mesh will increase the rigidity of the film making it easier to apply.

As already mentioned, the composites of the present invention are surprisingly non-oily and non-greasy to the touch. Only small amounts of the oleaginous material are released from the composites of the invention by squeezing, compared with the proportionately higher quantities of oil which are released from the sponges disclosed in WO-A-9101945. At least 90% by weight of the composites according to the present invention may be the stable, non-exuding oil phase. Insoluble protein - oil composites containing up to 70% by weight of dispersed oil feel non-oily when touched or gently squeezed.

The use of insoluble protein to form the matrix offers a number of surprising advantages over the use of soluble polymers as described in JP-A-55084167. First, the composites formed from insoluble proteins have excellent structural integrity when wetted in the wound dressing environment. The composites according to the present invention do not absorb water to form a sticky ointment. Instead, the composites according to the present invention maintain their integrity thereby providing support and protection to the wound. The composites according to the present invention degrade slowly in the wound, thereby releasing active agents into the wound at a controlled rate over an extended period. The composites according to the present invention are non-adhesive and readily removable from the wound with minimum wound trauma.

The invention is now further described with reference to the following examples, and to the Figures, in which:
Figure 1 is a scanning electron micrograph of an insoluble collagen/oil sponge according to the invention, and
Figure 2 is a light micrograph of an insoluble collagen/oil film according to the invention.

### Example 1: Insoluble Collagen/Oil Sponge

### A. Preparation of fibrous collagen from hide

The insoluble collagen used in the emulsion preparation is preferably collagen which is pre-washed and rendered largely free of fat, non-collageneous proteins, polysaccharides and other carbohydrates as described in US-A-4614794 or US-A-4320201 or British Patent Spec. No. 1 204 438. The collagen is suspended in clean deionised pyrogen free water and homogenised to a fine fibrous suspension by passage through a homogenising system. Suitable homogenising systems are described in US-A-4320201. Homogenising may be continued until a desired degree of fibre division is achieved. This results in a preferred fibre size of between 0.01 and 10mm. The collagen can then be used in this form (as an aqueous slurry) or freeze dried and milled to form a dehydrated or partially hydrated mass of fibres.

### B. Preparation of Sponge

The following components were used to prepare an insoluble collagen:oil sponge at a ratio of 1:10

| | |
|---|---|
| Biomedical grade fibrous collagen powder | 1.2 g |
| Soluble collagen in 0.05 M acetic acid (4 mg/ml) | 150 ml (0.6g) |
| Acetic acid 0.05 M | 432 ml |
| Vegetable oil | 18 g |

The components were chilled to 4°C and placed in a Waring Blendor. The mixture was homogenised three times at high speed for 30 secs before degassing in a vacuum chamber at <5 torr. The emulsion was then poured into an aluminium tray (255 x 500 mm) to give a thickness of 4 mm. The sample was blast frozen at -30°C before freeze drying.

### Example 2: Collagen/oil sponge

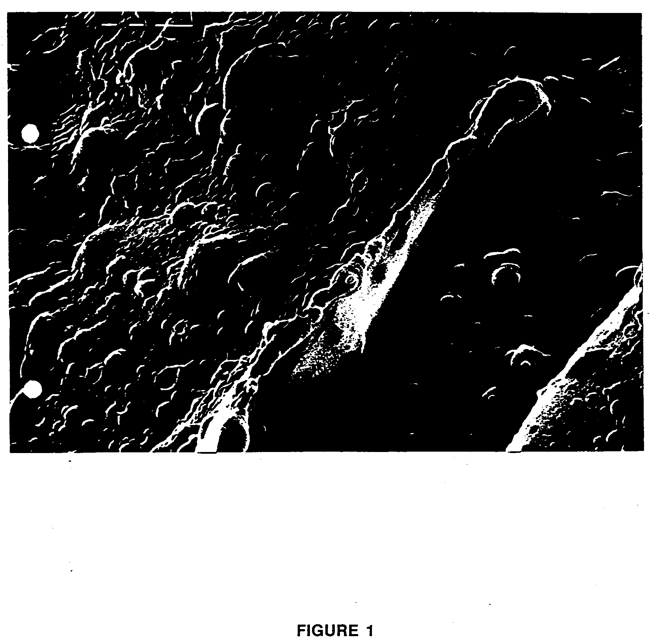

The procedure of example 1 was followed, but with an oil:collagen ratio of 2:1 (w/w). Figure 1 shows a scanning electron micrograph of the resulting sponge at a magnification of 1100x. Discrete oil droplets can clearly be seen under the surface of the collagen matrix.

### Example 3: Insoluble Collagen/Oil Film

The following components were used to prepare a collagen:oil film at a ratio of 1:2

| | |
|---|---|
| Biomedical grade fibrous collagen powder | 1.2 g |
| Soluble collagen in 0.05M acetic acid (4 mg/ml) | 150 ml (0.6g) |
| Acetic acid 0.05 M | 446.4 ml |
| Vegetable oil | 3.6 g |
| Glycerol | 0.12 g |

The components were chilled to 4°C and placed in a Waring Blendor. The mixture was homogenised three times at high speed for 30 secs before degassing in a vacuum chamber at <5 torr. The emulsion was then poured into a PVC tray (320 x 500 mm) and placed in a chamber and air dried at room temperature.

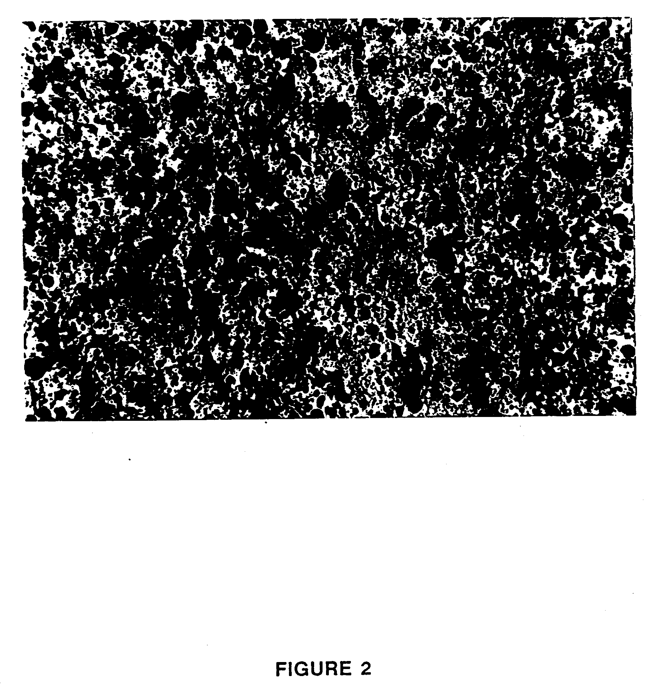

Figure 2 shows the resulting film at a magnification of 750x. As with Figure 1, discrete oil droplets (which have been stained with Sudan black) can be seen on the surface of the collagen.

### Example 4: Crosslinked Collagen/Oil Sponge

The following components were used to prepare an oil-collagen HMDI crosslinked sponge at a ratio of 1:2.

| | |
|---|---|
| Insoluble fibrous collagen | 3g |
| Mineral oil | 6g |
| HMDI | 0.06g |
| Deionised water | 594ml |

The components were chilled to 4°C and placed in a Waring Blendor. The mixture was homogenised at low speed for 30 secs while the HMDI cross linker (dispersed in a small volume of water and surfactant (marlophen)) was added. The homogenisation was continued at high speed for a further 60 secs. The slurry was then degassed in a vacuum chamber at <5torr. The emulsion was then poured into trays, blast frozen and freeze dried.

### Example 5: Collagen/Dispersible Oil Film

The following components were used to prepare a collagen:Labrafil M2125 CS oil film at a ratio of 1:2, using the procedure of Example 3:

| | |
|---|---|
| Biomedical grade fibrous collagen powder | 1.2 g |
| Soluble collagen in 0.05M acetic acid (4 mg/ml) | 150 ml (0.6g) |
| Acetic acid 0.05 M | 446.4 ml |
| Labrafil M2125 CS | 3.6 g |
| Glycerol | 0.12 g |

The use of a dispersible oil was found to produce a film which dried with less shrinkage and curling, as compared with the film of Example 3.

### Example 6: Film Extrusion

As an alternative to drying the emulsion on trays for film production, an extrusion technique can be used. Following the degassing stage, an emulsion prepared as in Example 2 was transferred to an extruder. The emulsion was maintained at a maximum temperature of 19 ± 4°C and extruded at 345 kPa (50 psi) onto a PTFE (polytetrafluoroethylene) coated glass fibre belt. The film was then batch dried on the conveyor at 45°C for approximately 20 mins.

### Example 7: Collagen/Oil Microspheres

The following components were used to prepare collagen/oil microspheres at a ratio of 1:1.

| | |
|---|---|
| Insoluble fibrous collagen | 1.8g |
| Acetic acid (0.01M) | 598.2ml |
| Mineral oil | 1.8g |

The components were chilled to 4°C and placed in a Waring Blendor. The mixture was homogenised at high speed for a total of 90 secs. The slurry was then degassed in a vacuum chamber at <5torr. The emulsion was then used to prepare microspheres by the method described in US Patent 4,837,285 and lyophilised.

### Example 8: Medicated Collagen-Alginate/Oil Film for the Treatment of Periodontitis

The following components were used to prepare a chlorhexidine medicated collagen-alginate/oil film for the treatment of periodontitis. Collagen-alginate : oil ratio is 1:5.

| | |
|---|---|
| Fibrous insoluble collagen | 1.62g |
| Sodium alginate | 0.18g |
| Chlorhexidine digluconate | 1.8g |
| Vegetable oil (Sesame oil) | 1.5g |
| 0.05M acetic acid | 598.5ml |
| Glycerol | 0.12g |

The sodium alginate was dissolved in the acetic acid containing the glycerol and the chlorhexidine was added to this solution during homogenisation in a Waring Blendor at low speed. The collagen and oil were then added and the components were homogenised at high speed for a total of 90 secs. The emulsion was then degassed in a vacuum chamber at <5torr before pouring the emulsion into a PVC tray (320mm x 500mm) and air drying at room temperature. The resulting medicated film was then cut into strips 2 x 10mm.

The above examples are intended for the purpose of illustration only.

## Claims

1. A process for preparing a composite comprising an insoluble protein matrix and an oleaginous material, said process comprising the steps of mixing a protein, the oleaginous material and water to form an emulsion of said oleaginous material in an aqueous dispersion of the protein, and subsequently drying the emulsion.

2. A process according to claim 1, wherein the mixing of the protein, oleaginous material and water is carried out at a temperature at which the oleaginous material is liquid.

3. A process according to claim 2, wherein the oleaginous material is a mineral oil or a vegetable oil.

4. A process according to any preceding claim, wherein the emulsion is dried as a thin layer to form a film.

5. A process according to any of claims 1 to 3, wherein the emulsion is frozen and then freeze dried, to form an insoluble protein based sponge.

6. A process according to any preceding claim, wherein the weight ratio of oleaginous material to insoluble protein in the emulsion is in the range 0.001:1 to 100:1.

7. A process according to any preceding claim further comprising the step of adding a cross-linking agent to the emulsion.

8. A process according to any preceding claim wherein the protein comprises an insoluble fibrous protein.

9. A process according to claim 8, wherein the insoluble fibrous protein is selected from insoluble collagen, keratin, fibrin, or elastin.

10. A process according to claim 9, wherein the insoluble fibrous protein is predominantly comprised of acid-swollen collagen.

11. A sponge wherein the material of the sponge comprises a composite of an insoluble protein and an oleaginous material distributed as microdroplets throughout the material of the sponge, the weight ratio of oleaginous material to insoluble protein in the material of the sponge being from 0.001:1 to 25:1.

12. A sponge according to claim 11, wherein the oleaginous material is an oil at 25°C.

13. A sponge according to claim 11 or 12 which is heteromorphic.

14. A film comprising a composite of an insoluble protein and an oleaginous material, the oleaginous material being distributed as microdroplets throughout the film, and the weight ratio of oleaginous material to protein being from 0.001:1 to 20:1.

15. A film according to claim 14, wherein the oleaginous material is an oil at 25°C.

16. A film according to claim 14 or 15 which is perforated or reticulated.

17. A film according to claim 14, 15 or 16 which further comprises a pharmaceutically active agent selected from the group consisting of steroids, antibiotics, antiseptics and peptide hormones and growth factors.

18. Use of a film according to claim 17 for the preparation of a medicated implant for use in the treatment of periodontal disease.

## Patentansprüche

1. Ein Verfahren zur Herstellung eines Verbundmaterials, das eine unlösliche Proteinmatrix und ein ölartiges Material umfaßt, wobei das Verfahren die Schritte umfaßt, daß ein Protein, das ölartige Material und Wasser vermischt werden, um eine Emulsion des ölartigen Materials in einer wässrigen Dispersion des Proteins zu bilden, und anschließend die Emulsion getrocknet wird.

2. Ein Verfahren nach Anspruch 1, dadurch gekennzeichnet, daß das Mischen des Proteins, ölartigen Materials und Wassers bei einer Temperatur durchgeführt wird, bei der das ölartige Material flüssig ist.

3. Ein Verfahren nach Anspruch 2, dadurch gekennzeichnet, daß das ölartige Material ein Mineralöl oder ein Pflanzenöl ist.

4. Ein Verfahren nach einem vorangehenden Anspruch, dadurch gekennzeichnet, daß die Emulsion als eine dünne Schicht getrocknet wird, um einen Film zu bilden.

5. Ein Verfahren nach einem der Ansprüche 1 bis 3, dadurch gekennzeichnet, daß die Emulsion gefroren und anschließend gefriergetrocknet wird, um einen Schwamm auf der Basis des unlöslichen Proteins zu bilden.

6. Ein Verfahren nach einem vorangehenden Anspruch, dadurch gekennzeichnet, daß das Gewichtsverhältnis von ölartigem Material zu unlöslichem Protein in der Emulsion im Bereich von 0,001:1 bis 100:1 liegt.

7. Ein Verfahren nach einem vorangehenden Anspruch, weiter gekennzeichnet durch den Schritt, daß ein Vernetzungsmittel zur Emulsion zugegeben wird.

8. Ein Verfahren nach einem vorangehenden Anspruch, dadurch gekennzeichnet, daß das Protein ein unlösliches faserartiges Protein umfaßt.

9. Ein Verfahren nach Anspruch 8, dadurch gekennzeichnet, daß das unlösliche faserartige Protein ausgewählt ist aus unlöslichem Kollagen, Keratin, Fibrin oder Elastin.

10. Ein Verfahren nach Anspruch 9, dadurch gekennzeichnet, daß das unlösliche faserartige Protein überwiegend aus säuregequollenem Kollagen besteht.

11. Ein Schwamm, dadurch gekennzeichnet, daß das Material des Schwammes ein Verbundmaterial aus einem unlöslichen Protein und einem ölartigen Material umfaßt, verteilt als Mikrotröpfchen im gesamten Material des Schwammes, wobei das Gewichtsverhältnis von ölartigem Material zu unlöslichem Protein im Material des Schwammes von 0,001:1 bis 25:1 beträgt.

12. Ein Schwamm nach Anspruch 11, dadurch gekennzeichnet, daß das ölartige Material ein Öl bei 25°C ist.

13. Ein Schwamm nach Anspruch 11 oder 12, dadurch gekennzeichnet, daß er heteromorph ist.

14. Ein Film, dadurch gekennzeichnet, daß er ein Verbundmaterial aus einem unlöslichen Protein und einem ölartigen Material umfaßt, wobei das ölartige Material als Mikrotröpfchen im gesamten Film verteilt ist und das Gewichtsverhältnis von ölartigem Material zu Protein von 0,001:1 bis 20:1 beträgt.

15. Ein Film nach Anspruch 14, dadurch gekennzeichnet, daß das ölartige Material ein Öl bei 25°C ist.

16. Ein Film nach Anspruch 14 oder 15, dadurch gekennzeichnet, daß dieser perforiert oder als Netz ausgebildet ist.

17. Ein Film gemäß Anspruch 14, 15 oder 16, welcher ferner einen pharmazeutisch aktiven Wirkstoff umfaßt, ausgewählt aus der Gruppe bestehend aus Steroiden, Antibiotika, Antispetika und peptiden Hormonen und Wachstumsfaktoren.

18. Verwendung eines Films gemäß Anspruch 17 zur Herstellung eines medizinischen Implantats zur Verwendung bei der Behandlung einer periodontalen Krankheit.

## Revendications

1. Procédé de préparation d'un matériau composite comprenant une matrice de protéine insoluble et un produit oléagineux, ledit procédé comprenant les étapes de mélanger une protéine, le produit oléagineux et de l'eau pour former une émulsion dudit produit oléagineux dans une dispersion aqueuse de la protéine, et de sécher ensuite l'émulsion.

2. Procédé selon la revendication 1, dans lequel le mélange de la protéine, du produit oléagineux et de l'eau est réalisé à une température à laquelle le produit oléagineux est liquide.

3. Procédé selon la revendication 2, dans lequel le produit oléagineux est une huile minérale ou une huile végétale.

4. Procédé selon l'une quelconque des revendications précédentes, dans lequel l'émulsion est séchée en une couche mince pour former un film.

5. Procédé selon l'une quelconque des revendications 1 à 3, dans lequel l'émulsion est congelée et ensuite lyophilisée, pour former une éponge à base de protéine insoluble.

6. Procédé selon l'une quelconque des revendications précédentes, dans lequel le rapport en poids du produit oléagineux à la protéine insoluble dans l'émulsion est dans la gamme 0,001:1 à 100:1.

7. Procédé selon l'une quelconque des revendications précédentes, comprenant en outre l'étape d'addition d'un agent de réticulation à l'émulsion.

8. Procédé selon l'une quelconque des revendications précédentes, dans lequel la protéine comprend une protéine fibreuse insoluble.

9. Procédé selon l'une quelconque des revendications précédentes, dans lequel la protéine fibreuse insoluble est choisie parmi le collagène insoluble, la kératine, la fibrine ou l'élastine.

10. Procédé selon la revendication 9, dans lequel la protéine fibreuse insoluble est constituée d'une manière prédominante de collagène gonflé dans un acide.

11. Eponge dans laquelle le matériau de l'éponge comprend un composite d'une protéine insoluble et d'un produit oléagineux réparti sous forme de microgouttelettes partout dans le matériau de l'éponge, le rapport en poids du produit oléagineux à la protéine insoluble dans le matériau étant de 0,001:1 à 25:1.

12. Eponge selon la revendication 11, dans laquelle le produit oléagineux est une huile à 25°C.

13. Eponge selon la revendication 11 ou 12 qui est hétéromorphe.

14. Film comprenant un composite d'une protéine insoluble et d'un produit oléagineux, le produit oléagineux étant réparti en microgouttelettes partout dans le film, et le rapport en poids du produit oléagineux à la protéine étant de 0,001:1 à 20:1.

15. Film selon la revendication 14, dans lequel le produit oléagineux est une huile à 25°C.

16. Film selon la revendication 14 ou 15 qui est perforé ou réticulé.

17. Film selon la revendication 14, 15 ou 16 qui comprend en outre un agent pharmaceutiquement actif choisi parmi le groupe constitué de stéroides, d'antibiotiques, d'antiseptiques et des hormones peptidiques et de facteurs de croissance.

18. Utilisation d'un film selon la revendication 17 pour la préparation d'un implant médicamenté pour l'utilisation dans le traitement de la périodontite.
